(19) Europäisches Patentamt
European Patent Office
Office européen des brevets

(11) Veröffentlichungsnummer: **0 332 029**
**A2**

(12) **EUROPÄISCHE PATENTANMELDUNG**

(21) Anmeldenummer: **89103538.8**

(22) Anmeldetag: **01.03.89**

(51) Int. Cl.4: **C07D 487/04 , A01N 43/90 , //(C07D487/04,249:00,239:00)**

(30) Priorität: **11.03.88 DE 3808122**

(43) Veröffentlichungstag der Anmeldung:
**13.09.89 Patentblatt 89/37**

(84) Benannte Vertragsstaaten:
**BE CH DE FR GB IT LI NL**

(71) Anmelder: **BAYER AG**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Babczinski, Peter, Dr.**
**In der Lohrenbeck 11**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Harry, Dr.**
**Unterdorfstrasse 6A**
**D-4000 Düsseldorf 31(DE)**

(54) **Triazolopyrimidin-2-sulfonamide.**

(57) Die Erfindung betrifft neue Triazolopyrimidin-2-sulfonamide der Formel (I),

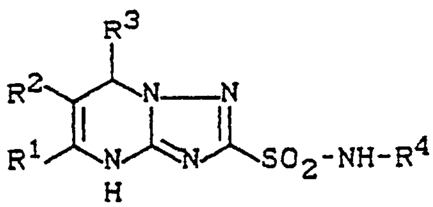

(I)

in welcher
$R^1$, $R^2$ und $R^3$ unabhängig voneinander jeweils für Wasserstoff oder für gegebenenfalls substituiertes Alkyl stehen und
$R^4$ für jeweils gegebenenfalls substituiertes Aryl oder Heteroaryl steht,
Verfahren zu deren Herstellung sowie deren Verwendung als Herbizide.



Xerox Copy Centre

## Triazolopyrimidin-2-sulfonamide

Die Erfindung betrifft neue Triazolopyrimidin-2-sulfonamide, ein Verfahren zu ihrer Herstellung und ihre Verwendung als Herbizide.

Es ist bekannt, daß bestimmte Triazolopyrimidin-2-sulfonamide, wie beispielsweise die Verbindung 5,7-Dimethyl-N-(2-methoxycarbonylphenyl)-1,2,4-triazolo[1,5-a]-pyrimidin-2-sulfonamid herbizide Eigenschaften besitzen (vgl. z. B. EP 142 152).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Triazolopyrimidin-2-sulfonamide der allgemeinen Formel (I),

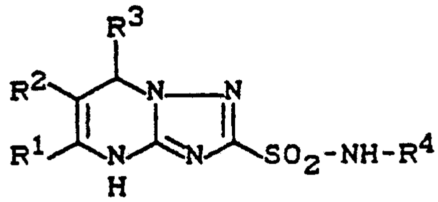

(I)

in welcher
$R^1$, $R^2$ und $R^3$ unabhängig voneinander jeweils für Wasserstoff oder für gegebenenfalls substituiertes Alkyl stehen und
$R^4$ für jeweils gegebenenfalls substituiertes Aryl oder Heteroaryl steht,
gefunden.

Die Verbindungen der Formel (I) stehen im Gleichgewicht mit den tautomeren Verbindungen der Formel (Ia),

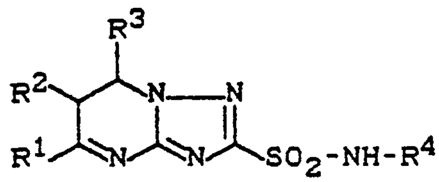

(Ia)

in welcher
$R^1$, $R^2$, $R^3$ und $R^4$ die oben angegebene Bedeutung haben;
sowohl Verbindungen der Formel (I) als auch Verbindungen der Formel (Ia) ebenso wie deren Gemische werden erfindungsgemäß beansprucht.

Weiterhin wurde gefunden, daß man die neuen Triazolopyrimidin-2-sulfonamide der allgemeinen Formel (I),

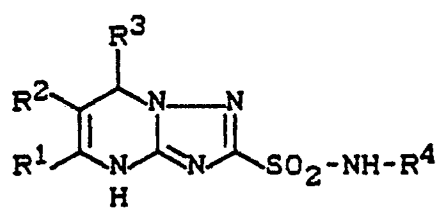

(I)

in welcher
$R^1$, $R^2$ und $R^3$ unabhängig voneinander jeweils für Wasserstoff oder für gegebenenfalls substituiertes Alkyl stehen und
$R^4$ für jeweils gegebenenfalls substituiertes Aryl oder Heteroaryl steht,
sowie deren Tautomere der Formel (Ia) erhält, wenn man Aminotriazolysulfonamide der Formel (II),

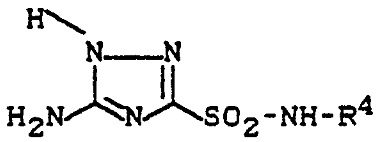

(II)

in welcher
$R^4$ die oben angegebene Bedeutung hat,
mit Ketoalkoholen der Formel (III),

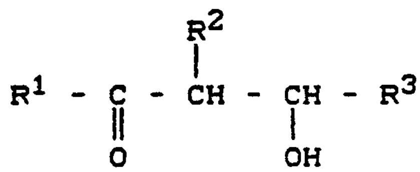

(III)

in welcher
$R^1$, $R^2$ und $R^3$ die oben angegebene Bedeutung haben,
gegebenenfalls in Gegenwart eines Verdünnungsmittels umsetzt.

Schließlich wurde gefunden, daß die neuen Triazolo-pyrimidin-2-sulfonamide der allgemeinen Formel (I) herbizide Eigenschaften besitzen.

Überraschenderweise zeigen die erfindungsgemäßen Triazolo-pyrimidin-2-sulfonamide der allgemeinen Formel (I) eine erheblich bessere herbizide Wirksamkeit gegen Problemunkräuter als die aus dem Stand der Technik bekannten Triazolo-pyrimidin-2-sulfonamid-Derivate, wie beispielsweise das 5,7-Dimethyl-N-(2-methoxycarbonylphenyl)-1,2,4-triazolo-[1,5-a]-pyrimidin-2-sulfonamid, welche chemisch und wirkungsmäßig naheliegende Verbindungen sind.

Die erfindungsgemäßen Triazolo-pyrimidin-2-sulfonamide sind durch die Formel (I) allgemein definiert. Bevorzugt sind Verbindungen der Formel (I), bei welchen

$R^1$, $R^2$ und $R^3$ unabhängig voneinander jeweils für Wasserstoff, für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder ver schiedenen Halogenatomen, für jeweils geradkettiges oder verzweigtes Alkoxyalkyl oder Alkylthioalkyl mit jeweils 1 bis 4 Kohlenstoffatomen in den einzelnen Alkylteilen oder für jeweils gegebenenfalls im Arylteil einfach oder mehrfach, gleich oder verschieden substituiertes Aryloxyalkyl oder Arylthioalkyl mit jeweils 1 bis 4 Kohlenstoffatomen im geradkettigen oder verzweigten Alkylteil und 6 bis 10 Kohlenstoffatomen im Arylteil stehen, wobei als Substituenten in Frage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und

$R^4$ für gegebenenfalls einfach oder mehrfach, gleich oder verschieden substituiertes Aryl mit 6 bis 10 Kohlenstoffatomen oder für einen gegebenenfalls einfach oder mehrfach, gleich oder verschieden substituierten und/oder benzannellierten 5- bis 7-gliedrigen Heterocyclus mit 1 bis 3 Heteroatomen, insbesondere Stickstoff, Sauerstoff und/oder Schwefel steht, wobei als Substituenten jeweils in Frage kommen: Fluor, Chlor, Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl oder Halogenalkylcarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

Besonders bevorzugt sind Verbindungen der allgemeinen Formel (I), bei welcher

$R^1$, $R^2$ und $R^3$ unabhängig voneinander jeweils für Wasserstoff, Methyl, Ethyl, n- oder i-Propyl, n-, i-, s- oder

t-Butyl, für Chlormethyl, Dichlormethyl, Trichlormethyl, Fluormethyl, Difluormethyl, Trifluormethyl, Dichlorfluormethyl, Chlordifluormethyl oder Fluorchlormethyl, für Methoxymethyl, Ethoxymethyl, n- oder i-Propoxymethyl, Methylthiomethyl, Ethylthiomethyl, n- oder i-Propylthiomethyl oder für im Phenylteil gegebenenfalls ein- bis dreifach gleich oder verschieden substituiertes Phenoxymethyl oder Phenylthiomethyl stehen, wobei als Substituenten in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl und

$R^4$ für jeweils gegebenfalls ein- bis dreifach, gleich oder verschieden substituiertes Phenyl oder Naphthyl oder für einen gegebenenfalls ein- bis dreifach gleich oder verschieden substituierten und/oder benzannellierten Heterocyclus der Formel

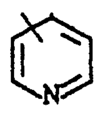 ; 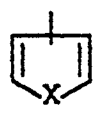 ; 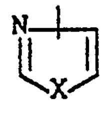 ; 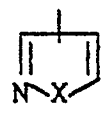 ; 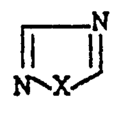

oder 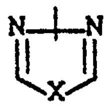 steht,

wobei X jeweils für Sauerstoff, Schwefel, eine NH-Gruppe oder eine N-$CH_3$-Gruppe steht und wobei als Phenyl-, Naphthyl- und Heterocyclus-Substituenten jeweils in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, n- oder i-Propoxy, Methylthio, Ethylthio, Acetyl, Propionyl, Methylsulfinyl, Methylsulfonyl, Trifluormethyl, Trifluormethoxy, Trifluormethylthio, Trifluormethylsulfinyl, Trifluormethylsulfonyl, Chloracetyl, Dichloracetyl, Trifluoracetyl, Phenyl, Phenoxy, Phenylthio, Benzoyl, Hydroxycarbonyl, Methoxycarbonyl, Ethoxycarbonyl, n- oder i-Propoxycarbonyl, Allyloxycarbonyl, Methoxymethoxycarbonyl, Ethoxyethoxycarbonyl, Hydroximinomethyl, Methoximinomethyl, Methoximinoethyl und Ethoximinoethyl.

Ganz besonders bevorzugt sind Verbindungen der allgemeinen Formel (I), bei welchen

$R^1$ und $R^3$ unabhängig voneinander für Wasserstoff, für Methyl, Ethyl, n- oder i-Propyl, n-, i-, s-oder t-Butyl, für Methoxymethyl, Ethoxymethyl, n- oder i-Propoxymethyl, für Methylthiomethyl, für Trifluormethyl, Trichlormethyl, für Di chlorfluormethyl, Chlordifluormethyl, für Phenoxymethyl oder Phenylthiomethyl stehen,

$R^2$ für Wasserstoff steht und

$R^4$ für jeweils gegebenenfalls ein- bis dreifach, gleich oder verschieden substituiertes Phenyl, α-Naphthyl oder β-Naphthyl steht oder für einen gegebenenfalls ein- bis dreifach gleich oder verschieden substituierten und/oder benzannellierten Heterocyclus der Formel

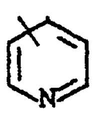 ; 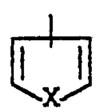 ; 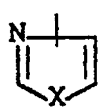 oder 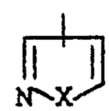 steht,

wobei X jeweils für Sauerstoff, Schwefel, eine NH-Gruppe oder eine N-$CH_3$-Gruppe steht und wobei als Phenyl-, Naphthyl- und Heterocyclus-Substituenten jeweils infrage kommen: Fluor, Chlor, Brom, Iod, Cyano, Nitro, Hydroxy, Methyl, Ethyl, Methoxy, Ethoxy, n- oder i-Propoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

Insbesondere bevorzugt sind Verbindungen der Formel (I), bei welchen

$R^1$ für Methyl oder Methoxymethyl steht,

$R^2$ für Wasserstoff steht,

$R^3$ für Wasserstoff, Methyl oder Methoxymethyl steht und

$R^4$ für gegebenenfalls ein- bis dreifach, gleich oder verschieden substituiertes Phenyl steht, wobei als Substituenten in Frage kommen: Fluor, Chlor, Brom, Cyano, Nitro, Hydroxy, Methyl, Ethyl, Methoxy, Ethoxy, n- oder i-Propoxy, Methylthio, Acetyl, Trifluormethyl, Trifluormethoxy, Trifluormethylthio, Phenyl, Phenoxy,

Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

Im einzelnen seien außer den bei den Herstellungsbeispielen genannten Verbindungen die folgenden Triazolopyrimidin-2-sulfonamide der allgemeinen Formel (I) genannt:

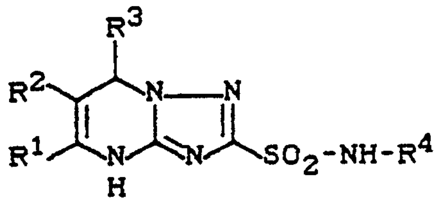

(I)

| $R^1$ | R2 | R3 | R4 |
|---|---|---|---|
| $CH_3$ | H | $CH_3$ | 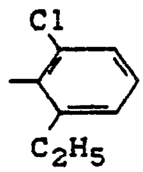 |

| $R^1$ | R2 | R3 | R4 |
|---|---|---|---|
| $CH_3$ | H | $CH_3$ | 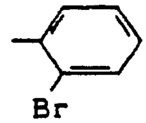 |
| $CH_3$ | H | $CH_3$ | 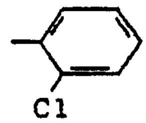 |
| $CH_3$ | H | $CH_3$ | 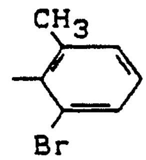 |
| $CH_3$ | H | $CH_3$ | 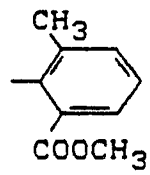 |
| $CH_3$ | H | $CH_3$ | 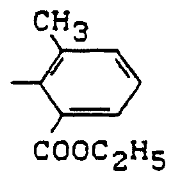 |
| $CH_3$ | H | $CH_3$ | 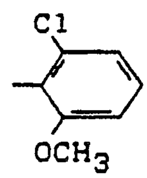 |
| $CH_3$ | H | $CH_3$ | 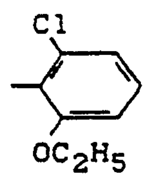 |
| $CH_3$ | H | $CH_3$ | 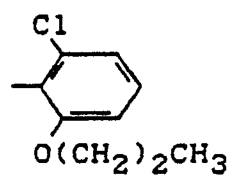 |

| $R^1$ | R2 | R3 | R4 |
|---|---|---|---|
| $CH_3$ | H | $CH_3$ | 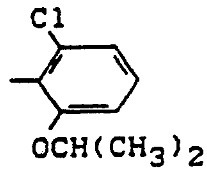 |
| $CH_3$ | H | $CH_3$ | 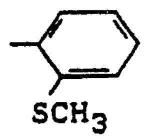 |
| $CH_3$ | H | $CH_3$ | 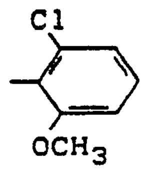 |
| $CH_3$ | H | $CH_3$ | 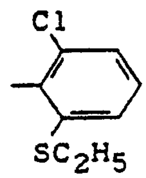 |
| $CH_3$ | H | $CH_3$ | 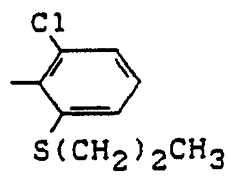 |
| $CH_3$ | H | $CH_3$ | 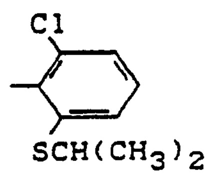 |
| $CH_3$ | H | $CH_3$ | 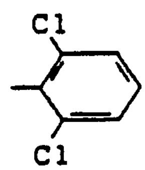 |
| $CH_3$ | H | $CH_3$ | 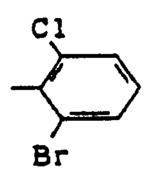 |

| $R^1$ | R2 | R3 | R4 |
|---|---|---|---|
| $CH_3$ | H | $CH_3$ | 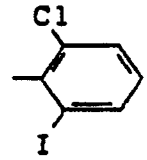 |
| $CH_3$ | H | $CH_3$ | 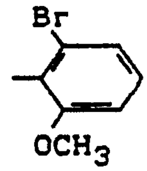 |
| $CH_3$ | H | $CH_3$ | 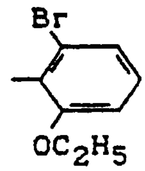 |
| $CH_3$ | H | $CH_3$ | 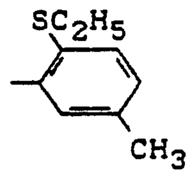 |
| $CH_3$ | H | $CH_3$ | 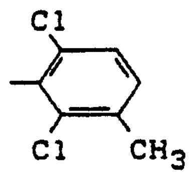 |
| $CH_3$ | H | $CH_3$ | 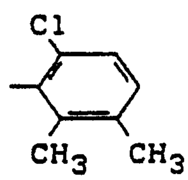 |
| $CH_3$ | H | H | 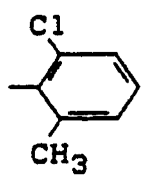 |
| $CH_3$ | H | H | 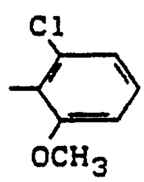 |

| $R^1$ | R2 | R3 | R4 |
|---|---|---|---|
| $CH_3$ | H | H | 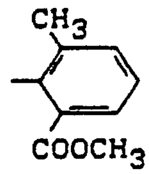 |
| $CH_3$ | H | H | 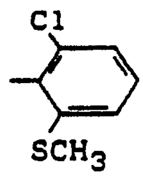 |
| $CH_3$ | H | H | 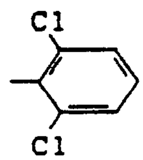 |
| $CH_3$ | H | H | 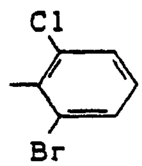 |
| $CH_3$ | H | H | 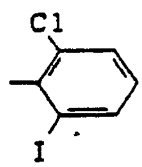 |
| $CH_3$ | H | $CH_3O-CH_2-$ | 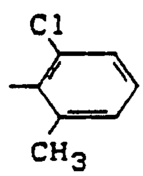 |
| $CH_3$ | H | $CH_3O-CH_2-$ | 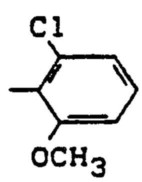 |
| $CH_3$ | H | $CH_3O-CH_2-$ | 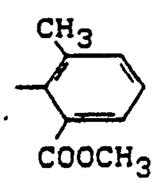 |

| $R^1$ | R2 | R3 | R4 |
|---|---|---|---|
| $CH_3$ | H | $CH_3O-CH_2-$ | 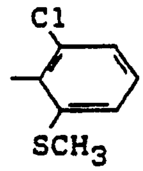 |
| $CH_3$ | H | $CH_3O-CH_2-$ | 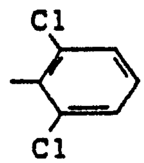 |
| $CH_3$ | H | $CH_3O-CH_2-$ | 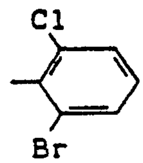 |
| $CH_3$ | H | $CH_3O-CH_2-$ | 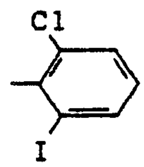 |
| $CH_3$ | H | $CH_3$ | 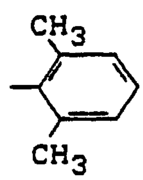 |
| $CH_3$ | H | $CH_3$ | 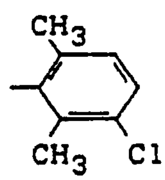 |
| $CH_3$ | H | $CH_3$ | 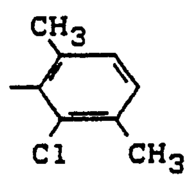 |
| $CH_3$ | H | $CH_3O-CH_2-$ | 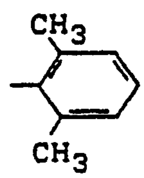 |

| $R^1$ | R2 | R3 | R4 |
|---|---|---|---|
| $CH_3$ | H | $CH_3O-CH_2-$ | 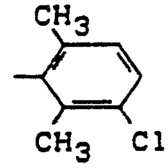 |
| $CH_3$ | H | $CH_3O-CH_2-$ | 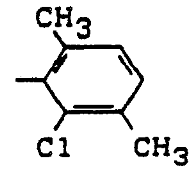 |
| $CH_3$ | H | H | 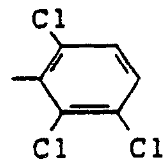 |

Verwendet man beispeilsweise 5-Amino-3-(2,6-dichlorphenylaminosulfonyl)-1,2,4-triazol und Pentan-4-ol-2-on als Ausgangsstoffe, so läßt sich der Reaktionsablauf des erfindungsgemäßen Verfahrens durch das folgende Formelschema darstellen:

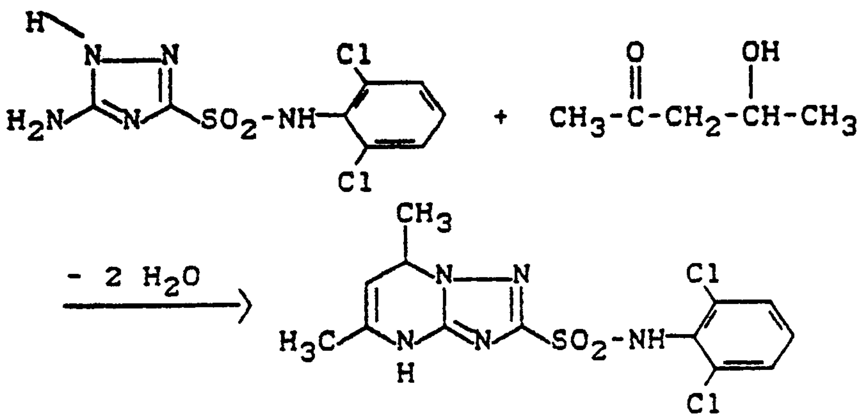


Die zur Durchführung des erfindungsgemäßen Verfahrens (a) als Ausgangsstoffe benötigten Aminotriazolylsulfonamide sind durch die Formel (II) allgemein definiert. In dieser Formel (II) steht $R^4$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diesen Substituenten genannt wurden. In der Formel (II) steht $R^4$ ganz besonders bevorzugt für diejenigen Reste, die im Zusammenhang mit der Beschreibung der erfindungsgemäßen Stoffe der Formel (I) als ganz besonders bevorzugt für diese Substituenten genannt wurden.

Die Aminotriazolylsulfonamide der Formel (II) sind teilweise bekannt (vgl. z. B. EP -A 244 844).

Man erhält sie, wenn man 3-Amino-5-benzylthio-1,2,4-triazol der Formel (IVa)

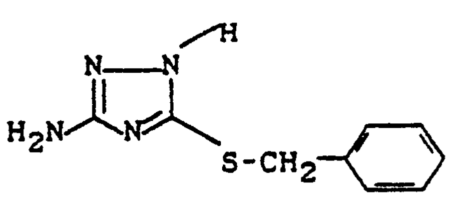

(IVa),

(vgl. z.B. J. Heterocycl. Chem 12, 1187 [1975] oder EP 142 152) welches im tautomeren Gleichgewicht vorliegt mit der entsprechenden 5-Amino-3-benzylthio-Verbindung der Formel (IVb)

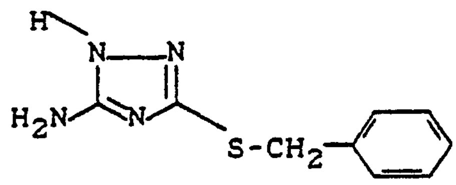

(IVb),

zunächst in einer 1. Stufe mit Chlorameisensäurephenylester in Gegenwart eines Verdünnungsmittels wie beispielsweise Pyridin bei Temperaturen zwischen -20 °C und +20 °C umsetzt und das so erhältliche 1-Phenoxycarbonyl-3-benzylthio-5-amino-1,2,4-triazol der Formel (V)

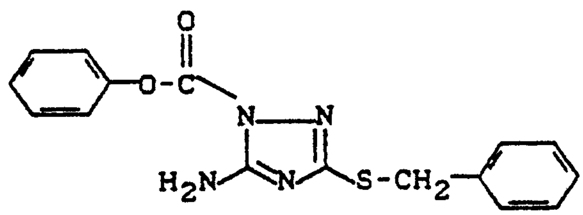

(V)

in einer 2. Stufe mit elementarem Chlor in Gegenwart von Wasser und in Gegenwart eines Verdünnungsmittels, wie beispeilsweise Chloroform sowie in Gegenwart eines Reaktionshilfmittels wie beispielsweise Eisessig bei Temperaturen zwischen -20 °C und +20 °C umsetzt und das derart erhältliche 5-Amino-1-phenoxycarbonyl-1,2,4-triazol-3-yl-sulfonylchlorid der Formel (VI)

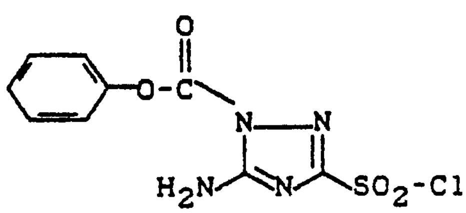

(VI)

in einer 3. Stufe mit Aminen der Formel (VII),

$R^4 - NH_2$ (VII)

in welcher

$R^4$ die oben angegebene Bedeutung hat,

gegebenenfalls in Gegenwart eines Verdünnungsmittels wie beispeilsweise Dichlormethan sowie in Gegenwart eines Säurebindemittels wie beispielsweise Pyridin und gegebenenfalls in Gegenwart eines Reaktionshilfsmittels wie beispielsweise 4-Dimethylaminopyridin bei Temperaturen zwischen 0 °C und 60 °C umsetzt und anschließend in einer 4. Stufe aus den so erhältlichen 5-Amino-1-phenoxycarbonyl-1,2,4-triazol-3-yl-sulfonamiden der Formel (VIII),

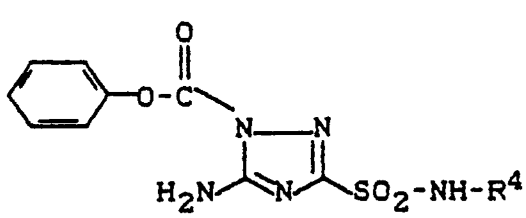

(VIII)

in welcher

$R^4$ die oben angegebene Bedeutung hat,

mit wäßriger Natronlauge gegebenenfalls in Gegenwart eines Verdünnungsmittels wie beispielsweise

Ethanol bei Temperaturen zwischen 0 °C und 40 °C die Phenoxycarbonyl-Schutzgruppe in der 1-Position des Triazolringes wieder abspaltet.

Amine der Formel (VII) sind allgemein bekannte Verbindungen der organischen Chemie.

Die zur Durchführung des erfindungsgemäßen Verfahrens weiterhin als Ausgangsstoffe benötigten Ketoalkohole sind durch die Formel (III) allgemein definiert. In dieser Formel (III) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Ketoalkohole der Formel (III) sind allgemein bekannte Verbindungen oder erhältlich in Analogie zu allgemein bekannten Verfahren.

Als Verdünnungsmittel zur Durchführung des erfindungsgemäßen Verfahrens kommen inerte organische Lösungsmittel in Frage. Mit besonderem Vorzug verwendet man polare organische Lösungsmittel, beispielsweise höhersiedende Alkohole wie Ethylenglykolmonoethylether, Ethanol, Propanol oder Butanol oder Carbonsäuren wie beispielsweise Essigsäure.

Die Reaktionstemperaturen können bei der Durchführung des erfindungsgemäßen Verfahrens in einem größeren Bereich variiert werden.Im allgemeinen arbeitet man bei Temperaturen zwischen 20 °C und 200 °C, vorzugsweise bei Temperaturen zwischen 50 °C und 150 °C.

Zur Durchführung des erfindungsgemäßen Verfahrens setzt man pro Mol an Aminotriazolylsulfonamid der Formel (II) im allgemeinen 1,0 bis 3,0 Mol, vorzugsweise 1,0 bis 2,0 Mol an Ketoalkohol der Formel (III) ein. Die Reaktionsdurchführung, Aufarbeitung und Isolierung der Reaktionsprodukte der Formel (I) erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur

selektiven Unkrautbekämpfung in einjährigen Kulturen eingesetzt werden.

Die erfindungsgemäßen Verbindungen der Formel (I) eignen sich besonders zur Bekämpfung mono- und dikotyler Unkräuter in monokotylen Kulturen, wie beispielsweise Weizen, im Vor- und im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, in Frage.
Auch Mischungen mit 2,4-Dichlorphenoxyessigsäure (2,4-D); 4-(2,4-Dichlorphenoxy)-buttersäure (2,4-DB); 2,4-Di- chlorphenoxypropionsäure (2,4-DP); Methyl-6,6-dimethyl-2,4-dioxo-3-[1-(2-propenyloxyamino)-butyliden]-cyclohexancarbonsäure (ALLOXYDIM); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP), N-Methyl-2-(1,3-benzthiazol-2-yloxy)-acetanilid

(MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 3,5,6-Trichlor-2-pyridyloxyessigsäure (TRICLOPYR) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

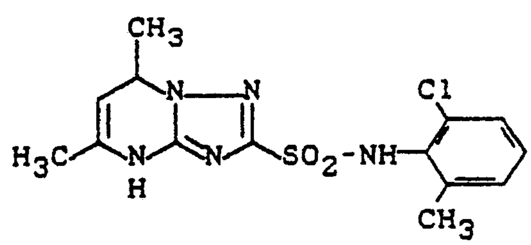

5 g (0.0174 Mol) 5-Amino-3-(2-chlor-6-methyl-phenylaminosulfonyl)-1,2,4-triazol und 3,0 g (0.0294 Mol) Pentan-4-ol-2-on (vgl. z. B. Chem. Ber. 100, 605 [1967]) werden in 50 ml Dioxan unter einer Stickstoffschutzgasatmosphäre 40 Stunden auf Rückflußtemperatur erhitzt, abgekühlt, im Vakuum eingeengt und chromatographisch gereinigt (Kieselgel; Laufmittel: Dichlormethan/Aceton 4 : 1). Der so erhältliche Feststoff wird mit wenig Essigester verrührt, abgesaugt und getrocknet.

Man erhält 1,8 g (30 % der Theorie) an 5,7-Dimethyl-N-(2-chlor-6-methylphenyl)-1,2,4-triazolo[1,5a]-[4H,7H]-dihydropyrimidin-2-sulfonamid vom Schmelzpunkt 211 °C - 214 °C.

In entsprechender Weise und gemäß den allgemeinen Angaben zur Herstellung erhält man die folgenden Triazolopyrimidin-2-sulfonamide der allgemeinen Formel (I):

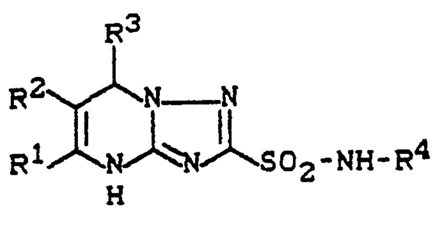

(I)

**Tabelle 1**

| Bsp.-Nr. | $R^1$ | $R^2$ | $R^3$ | $R^4$ | Schmelzpunkt /°C |
|---|---|---|---|---|---|
| 2 | H | H | $CH_3$ | 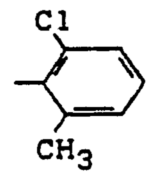 | 240 |
| 3 | $CH_3$ | H | $CH_3$ | 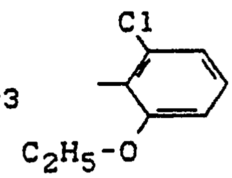 | 178 |
| 4 | $CH_3$ | H | $CH_3$ | 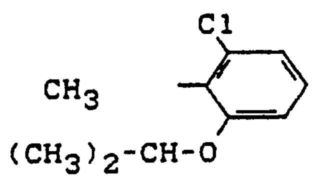 | 204 |

Herstellung der Ausgangsverbindungen

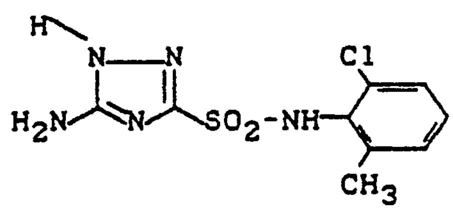

34,1 g (0.0837 Mol) 5-Amino-1-phenoxycarbonyl-1,2,4-triazol-3-yl-[N-(2-chlor-6-methyl-phenyl)]-sulfonamid in 350 ml Ethanol werden mit 14,9 g (0.168 Mol) 45 %iger wäßriger Natronlauge versetzt, eine Stunde bei Raumtemperatur gerührt, mit Eisessig angesäuert und im Vakuum eingeengt. Der Rückstand wird dreimal mit Wasser gewaschen und anschließend bei 80 °C im Vakuum getrocknet. Der so erhaltene Feststoff wird durch Verrühren mit Diethylether gereinigt.

Man erhält 13,5 g (56 % der Theorie) an 5-Amino-1,2,4-triazol-3-yl-[N-(2-chloro-6-methyl-phenyl)]-sulfonamid vom Schmelzpunkt 217 °C.

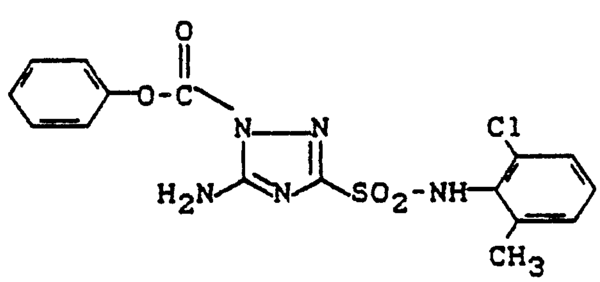

Zu 7,3 g (0.0922 Mol) absolutem Pyridin, 13 g (0.092 Mol) 2-Chlor-6-methylanilin und 1,1 g (0.009 Mol) 4-Di methylaminopyridin im 300 ml trockenem Dichlormethan gibt man 27,9 g (0.0922 Mol) 5-Amino-1-phenoxycarbonyl-1,2,4-triazol-3-yl-sulfochlorid, rührt eine Stunde bei Raumtemperatur, entfernt das Lösungsmittel im Vakuum, verrührt den öligen Rückstand mit Wasser, dekantiert und kristallisiert das verbleibende Öl aus Ethanol.

Man erhält 35,5 g (95 % der Theorie) an 5-Amino-1-phenoxycarbonyl-1,2,4-triazol-3-yl-[N-(2-chlor-6-methylphenyl)]-sulfonamid vom Schmelzpunkt 200 °C.

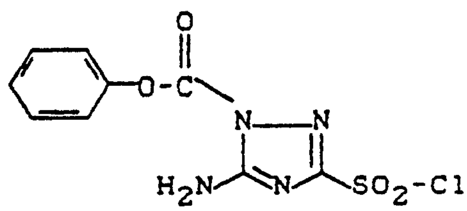

Zu 136,6 g (0,419 Mol) 5-Amino-1-phenoxycarbonyl-3-benzylthio-1,2,4-triazol in 1700 ml Chloroform gibt man 30 ml Eisessig und 15 ml Wasser und leitet durch diese Suspension bei -5 °C eine Stunde lang einen nicht getrockneten Chlorgasstrom, wobei sich eine klare Lösung bildet. Zur Aufarbeitung entfernt man das Lösungsmittel im Vakuum und verrührt den Rückstand mit 1 l Ether. Der etherunlösliche Feststoff wird abgesaugt und getrocknet.

Man erhält 101 g (80 % der Theorie) an 5-Amino-1-phenoxycarbonyl-1,2,4-triazol-3-yl-sulfochlorid vom Schmelzpunkt 164 °C.

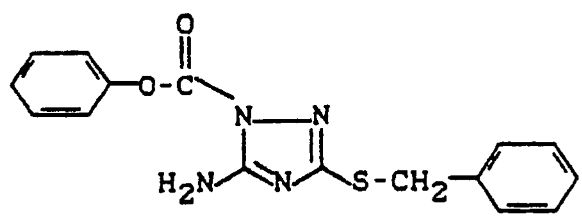

Zu 100 g (0.4854 Mol) 3-Amino-5-benzylthio-1,2,4-triazol (vgl. J. Het. Chem. 12, 1187 [1975]) in 700 ml absolutem Pyridin tropft man unter Kühlung 83,6 g (0.5339 Mol) Chlorameisensäurephenylester, so daß die Innentemperatur nicht über 5 °C ansteigt. Nach beendeter Zugabe rührt man weitere 60 Minuten bei 10 °C, gießt dann das Reaktionsgemisch in Eiswasser, saugt den ausgefallenen Feststoff ab und kristallisiert aus Ethanol um.

Man erhält 140 g (88,5 % der Theorie) an 5-Amino-3-benzylthio-1-phenoxycarbonyl-1,2,4-triazol vom Schmelz-punkt 285 °C (Zers.).

Anwendungsbeispiele

In den folgenden Anwendungsbeispielen wird die nachstehend aufgeführte Verbindung als Vergleichsubstanz eingesetzt:

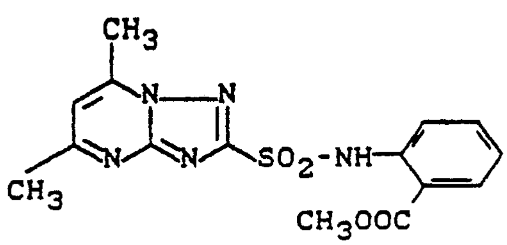

(A)

5,7-Dimethyl-N-(2-methoxycarbonyl-phenyl)-1,2,4-tria-zolo-[1,5-a]-pyrimidin-2-sulfonamid
(bekannt aus EP-A 142 152/Verbindung 10)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100% = totale Vernichtung

Eine deutliche Überlegenheit in der herbiziden Wirksamkeit gegen mono- und dikotyle Unkräuter gegenüber der Vergleichsverbindung (A) zeigt in diesem Test beispielsweise die Verbindung gemäß dem Herstellungsbeispiel (1).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der herbiziden Wirksamkeit gegen mono- und dikotyle Unkräuter gegenüber der Vergleichsverbindung (A) zeigt in diesem Test z. B. die Verbindung gemäß Herstellungsbeispiel (1).

**Ansprüche**

1. Triazolopyrimidin-2-sulfonamide der Formel (I),

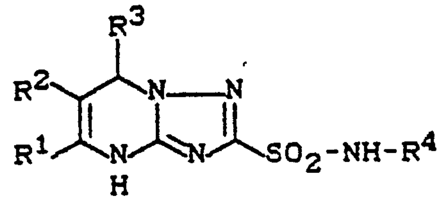

(I)

in welcher

$R^1$, $R^2$ und $R^3$ unabhängig voneinander jeweils für Wasserstoff oder für gegebenenfalls substituiertes Alkyl stehen und
$R^4$ für jeweils gegebenenfalls substituiertes Aryl oder Heteroaryl steht,
sowie deren Tautomere.

2. Triazolopyrimidin-2-sulfonamide der Formel (I) gemäß Anspruch 1, in welcher
$R^1$, $R^2$ und $R^3$ unabhängig voneinander jeweils für Wasserstoff, für geradkettiges oder verzweigtes Alkyl mit 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkoxyalkyl oder Alkylthioalkyl mit jeweils 1 bis 4 Kohlenstoffatomen in den einzelnen Alkylteilen oder für jeweils gegebenenfalls im Arylteil einfach oder mehrfach, gleich oder verschieden substituiertes Aryloxyalkyl oder Arylthioalkyl mit jeweils 1 bis 4 Kohlenstoffatomen im geradkettigen oder verzweigten Alkylteil und 6 bis 10 Kohlenstoffatomen im Arylteil stehen, wobei als Substituenten jeweils in Frage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl und
$R^4$ für gegebenenfalls einfach oder mehrfach, gleich oder verschieden substituiertes Aryl mit 6 bis 10 Kohlenstoffatomen oder für einen gegebenenfalls einfach oder mehrfach, gleich oder verschieden substituierten und/oder benzannellierten 5- bis 7-gliedrigen Heterocyclus mit 1 bis 3 Heteroatomen steht, wobei als Sub stituenten jeweils in Frage kommen: Fluor, Chlor, Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl oder Halogenalkylcarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

3. Triazolopyrimidin-2-sulfonamide der Formel (I) gemäß Anspruch 1, in welcher
$R^1$, $R^2$ und $R^3$ unabhängig voneinander jeweils für Wasserstoff, Methyl, Ethyl, n- oder i-Propyl, n-, i-, s- oder t-Butyl, für Chlormethyl, Dichlormethyl, Trichlormethyl, Fluormethyl, Di fluormethyl, Trifluormethyl, Dichlorfluormethyl, Chlordifluormethyl oder Fluorchlormethyl, für Methoxymethyl, Ethoxymethyl, n- oder i-Propoxymethyl, Methylthiomethyl, Ethylthiomethyl, n- oder i-Propylthiomethyl oder für im Phenylteil gegebenenfalls ein- bis dreifach gleich oder verschieden substituiertes Phenoxymethyl oder Phenylthiomethyl stehen, wobei als Substituenten in Frage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl und
$R^4$ für jeweils gegebenenfalls ein- bis dreifach, gleich oder verschieden substituiertes Phenyl oder Naphthyl oder für einen gegebenenfalls ein- bis dreifach gleich oder verschieden substituierten und/oder benzannellierten Heterocyclus der Formel

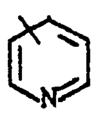 ; 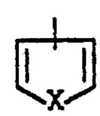 ; 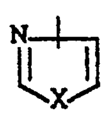 ; 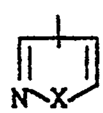 ; 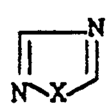

oder 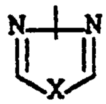 steht,

wobei X jeweils für Sauerstoff, Schwefel, eine NH-Gruppe oder eine $N-CH_3$-Gruppe steht und wobei als Phenyl-, Naphthyl- und Heterocyclus-Substituenten jeweils in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, n- oder i-Propoxy, Methylthio, Ethylthio, Acetyl, Propionyl, Methylsulfinyl, Methylsulfonyl, Trifluormethyl, Trifluormethoxy, Trifluormethylthio, Trifluormethylsulfinyl, Trifluormethylsulfonyl, Chloracetyl, Dichloracetyl, Trifluoracetyl, Phenyl, Phenoxy, Phenylthio, Benzoyl, Hydroxycarbonyl, Methoxycarbonyl, Ethoxycarbonyl, n- oder i-Propoxycarbonyl, Allyloxycarbonyl, Methoxymethoxycarbonyl, Ethoxyethoxycarbonyl, Hydroximinomethyl, Methoximinomethyl, Methoximinoethyl und Ethoximinoethyl.

4. Triazolopyrimidin-2-sulfonamide der Formel (I) gemäß Anspruch 1, in welcher
$R^1$ und $R^3$ unabhängig voneinander für Wasserstoff, für Methyl, Ethyl, n- oder i-Propyl, n-, i-, s- oder t-Butyl, für Methoxymethyl, Ethoxymethyl, n- oder i-Propoxymethyl, für Methylthiomethyl, für Trifluormethyl, Trichlormethyl, für Dichlorfluormethyl, Chlordi fluormethyl, für Phenoxymethyl oder Phenylthiomethyl stehen,
$R^2$ für Wasserstoff steht und
$R^4$ für jeweils gegebenenfalls ein- bis dreifach, gleich oder verschieden substituiertes Phenyl, α-Naphthyl oder β-Naphthyl steht oder für einen gegebenenfalls ein- bis dreifach gleich oder verschieden substituierten und/oder benzannellierten Heterocyclus der Formel

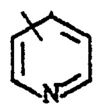 ; 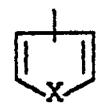 ; 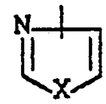 oder 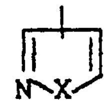 steht,

wobei X jeweils für Sauerstoff, Schwefel, eine NH-Gruppe oder eine $N\text{-}CH_3$-Gruppe steht und wobei als Phenyl-, Naphthyl- und Heterocyclus-Substituenten jeweils infrage kommen: Fluor, Chlor, Brom, Iod, Cyano, Nitro, Hydroxy, Methyl, Ethyl, Methoxy, Ethoxy, n- oder i-Propoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

5. Triazolopyrimidin-2-sulfonamide der Formel (I) gemäß Anspruch 1, in welcher
$R^1$ für Methyl oder Methoxymethyl steht,
$R^2$ für Wasserstoff steht,
$R^3$ für Wasserstoff, Methyl oder Methoxymethyl steht und
$R^4$ für gegebenenfalls ein- bis dreifach, gleich oder verschieden substituiertes Phenyl steht, wobei als Substituenten in Frage kommen: Fluor, Chlor, Brom, Cyano, Nitro, Hydroxy, Methyl, Ethyl, Methoxy, Ethoxy, n- oder i-Propoxy, Methylthio, Acetyl, Trifluormethyl, Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

6. Verfahren zur Herstellung von Triazolopyrimidin-2-sulfonamiden der allgemeinen Formel (I),

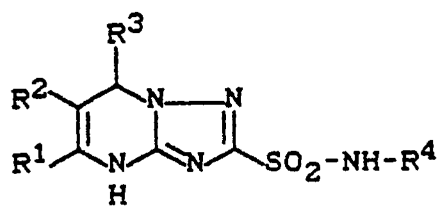

(I)

in welcher
$R^1$, $R^2$ und $R^3$ unabhängig voneinander jeweils für Wasserstoff oder für gegebenenfalls substituiertes Alkyl stehen und
$R^4$ für jeweils gegebenenfalls substituiertes Aryl oder Heteroaryl steht,
sowie deren Tautomeren, dadurch gekennzeichnet, daß man Aminotriazolylsulfonamide der Formel (II),

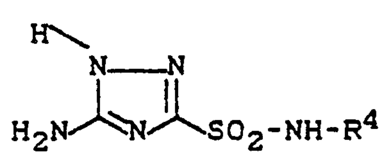

(II)

in welcher
$R^4$ die oben angegebene Bedeutung hat,

mit Ketoalkoholen der Formel (III),

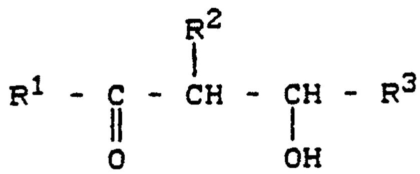 (III)

in welcher
$R^1$, $R^2$ und $R^3$ die oben angegebene Bedeutung haben,
gegebenenfalls in Gegenwart eines Verdünnungsmittels umsetzt.

7. Herbizide Mittel, gekennzeichnet durch einen Gehalt an mindestens einem Triazolopyrimidin-2-sulfonamid der Formel (I) gemäß den Ansprüchen 1 bis 6.

8. Verfahren zur Bekämpfung von Unkräutern, dadurch gekennzeichnet, daß man Triazolopyrimidin-2-sulfonamide der Formel (I) gemäß den Ansprüchen 1 bis 6 auf die Unkräuter und/oder ihren Lebensraum einwirken läßt.

9. Verwendung von Triazolopyrimidin-2-sulfonamiden der Formel (I) gemäß den Ansprüchen 1 bis 6 zur Bekämpfung von Unkräutern.

10. Verfahren zur Herstellung von herbiziden Mitteln, dadurch gekennzeichnet, daß man Triazolopyrimidin-2-sulfonamide der Formel (I) gemäß den Ansprüchen 1 bis 6 mit Streckmitteln und/oder oberflächenaktiven Substanzen vermischt.